# EUROPEAN PATENT SPECIFICATION

(11) **EP 4 251 120 B1**
(45) Date of publication and mention of the grant of the patent: **09.10.2024**
(21) Application number: 21810024.6
(22) Date of filing: 16.11.2021
(51) Int. Cl.: A61K 8/22, A61Q 19/00, A61Q 5/00, C09C 1/00

(54) **USE OF A PARTICULAR METAL OXIDE FOR THE PHOTOCONVERSION OF ORGANIC COMPOUNDS ON KERATIN MATERIALS**
VERWENDUNG EINES BESTIMMTEN METALLOXIDS ZUR PHOTOKONVERSION ORGANISCHER VERBINDUNGEN AUF KERATINMATERIALIEN
UTILISATION D'UN OXYDE MÉTALLIQUE PARTICULIER POUR LA PHOTOCONVERSION DE COMPOSÉS ORGANIQUES SUR DES MATIÈRES KÉRATINIQUES

(30) Priority: 24.11.2020 FR 2012072
(43) Date of publication of application: 04.10.2023
(73) Proprietor: L'OREAL, 75008 Paris (FR)
(72) Inventor: JEANNE-ROSE, Valérie, 93601 AULNAY-SOUS-BOIS (FR); SAMAIN, Henri, 94152 CHEVILLY-LARUE (FR); DELIGIANNAKIS, Yiannis, 45110 IOANNINA (GR); LOULOUDI, Maria, 45110 IOANNINA (GR)
(74) Representative: Casalonga
(86) International application number: PCT/EP2021/081788
(87) International publication number: WO 2022/112053

(56) References cited:
- FR-A1- 2 850 270
- FR-A1- 2 927 534
- JP-A- 2006 111 606
- US-A1- 2015 173 482

## Description

The present invention relates to the use of a particular metal oxide for the photoconversion of undesirable organic compounds capable of being present on keratin materials, in particular human keratin materials such as the skin, the scalp and keratin fibres such as the hair. This particular use makes it possible to eliminate the deposits of organic compounds at the surface of the keratin materials, or at the surface of objects in contact with said keratin materials.

The invention also relates to a method for the cosmetic treatment of keratin materials or of the surface of an object in contact with the keratin materials, using one such metal oxide.

The invention makes it possible in particular to reduce and/or slow down the oiling phenomena of the keratin materials.

Keratin materials have a tendency to lose some of their aesthetic qualities over time owing to the deposit or formation at their surface of undesirable organic compounds, which may originate from very diverse factors such as natural oiling (linked among others to the secretion of sebum), sweat, the elimination of scurf, pollution, humidity, etc. These may be compounds secreted by the human body (fatty substances such as sebum, protein derivatives, dead skin residues) or of external compounds which are deposited on the keratin materials, such as dirt, pollutants from vehicles or industry, etc.

The presence of these organic compounds on the keratin materials may give rise to discomfort and/or pose aesthetic problems. In particular, these compounds are detrimental to the visual appearance and to the feel of the keratin materials.

Thus for example natural oiling weighs down the hair, which then has a tendency to clump together. The hair is then more difficult to style, has an unpleasant greasy sheen and an unpleasant waxy feel. Similarly, the secretion of sebum by the skin gives the latter a greasy and shiny appearance considered to be particularly unattractive, especially on the face.

In order to eliminate the undesirable organic compounds, use is generally made of detergent compositions such as soaps, shower gels, shampoos, etc. This is because washing keratin materials with detergent compositions is very effective, and makes it possible to remove both the compounds secreted by the body and the dirt from the outside environment.

However, the beneficial effect of the washing rapidly wears off and the undesirable organic compounds are redeposited in a few days or even in a few hours, again generating the problems described above. Consequently, individuals wishing to avoid these problems have a tendency to increase the washing frequency, which often has the effect of stimulating the body secretions and increasing the scale thereof, and therefore of accelerating the rate of natural reoiling of the keratin materials after they have been washed.

Furthermore, these washes do not make it possible to carry out a regular removal as the undesirable compounds appear and to preserve a clean appearance of the keratin materials. In general, one waits for the undesirable compounds to accumulate sufficiently before washing the keratin materials. The washes must be repeated regularly and, between two washes, it is common for the individual to suffer from the presence of undesirable compounds.

Moreover, to perform these washes, it is necessary to have a source of water, preferably hot or warm water. The detergent compositions often contain large amounts of surfactants that may cause unpleasantness such as stinging on the scalp, skin or in the eyes.

In order to cleanse the keratin materials more rapidly and to avoid wetting them, it has already been proposed to use dry compositions such as "dry" shampoos, or disposable wipes. Cleansing with dry shampoo consists in spraying absorbent particles onto the hair and then in actively brushing the head of hair in order to remove the dirt. However, in general, complete removal of the dirt is very difficult to obtain. The results are not very satisfactory: the hair is dull, not very shiny and has a coarse feel. Furthermore, the use of disposable wipes, which comprise detergent compositions deposited on a textile support, poses environmental problems.

Solutions that are also known consist in eliminating from the keratin materials all or some of the undesirable compounds (including sebum, pollutants, etc.) by chemical oxidation. The principle consists in bringing together an oxidizing agent and the compounds to be eliminated on the hair. However, this technique is not satisfactory since its effect is immediate and cannot be continued over time given the absence of persistence of the oxidizing agent linked to its complete consumption during the reaction. Furthermore, the oxidizing agents used may have a harmful action on the keratin materials.

It is also known to eliminate from the keratin materials all or some of the foreign bodies or undesirable substances by photocatalysis in the presence of UV, in the presence of a catalyst such as for example titanium oxide. However, these photocatalysts have only a limited activity under irradiation in visible light. The photoconversion kinetics are often too slow, which means that the undesirable compounds accumulate faster than they are removed.

FR2850270 A1 discloses the use of a photocatalyst for reducing/eliminating undesirable organic compounds from the hair. The photocatalyst is for example V 205, CeO2. Nb2O5, WO3, Na4W10032, or Bi203. FR2927534 A1 discloses the use of a photocatalyst such as CaBi 204 for reducing/eliminating undesirable organic compounds from the hair. JP2006111606 A discloses the use of a photocatalyst such as titanium dioxide or tungsten oxide for the treatment of hair including an irradiation step. US2015173482 A1 discloses a comb coated with a layer of photocatalyst such as titanium dioxide, with which it is possible to remove organic residues from the hair.

The aim of the present invention is especially to solve the above problems.

More specifically, the present invention aims to provide a method for the cosmetic treatment of keratin materials, in particular human keratin materials, in order to reduce and/or slow down the oiling linked to the deposit of both natural organic compounds (in particular secreted by the body) and organic compounds from the outside environment. The invention very particularly makes it possible to prevent and/or reduce the deposits of sebum on the keratin materials.

The present invention also makes it possible to eliminate the organic compounds capable of being deposited at the surface of objects that come into contact with the keratin materials.

Following considerable research conducted in this matter, the applicant has found that these objectives, and others, could be achieved by using particles of a particular metal oxide as described above.

The present invention thus relates to the use, for preventing, reducing and/or eliminating the deposits of organic compounds at the surface of the keratin materials, in particular human keratin materials, or at the surface of objects in contact with said keratin materials, of solid particles comprising at least one metal oxide OM1 of formula AₐB_{b}Oₙ₋ₓ, in which:
A denotes a metal chosen from bismuth, calcium, sodium, lanthanum, barium, copper, tin, magnesium, zinc and silver;
B denotes a metal chosen from tungsten, vanadium, gallium, niobium and strontium;
a is an integer within the range of from 1 to 4;
b is an integer within the range of from 1 to 10;
n is an integer within the range of from 3 to 6; and
x is a decimal number of less than n, within the range of from 0.1 to 5.

The applicant has discovered that the particular OM1 oxides defined above very effectively catalyse the photodegradation of the organic compounds capable of being present on the keratin materials, with kinetics high enough to enable both the elimination of organic compounds already deposited and the degradation of such compounds as they appear, which makes it possible to reduce the deposits and to prevent the accumulation of new deposits.

The invention thus makes it possible to prevent and/or reduce the deposits of undesirable organic compounds capable of being present on the keratin materials, whether these are compounds originating from the outside environment (for example stains, dirt, pollutants), or compounds secreted by the body, in particular proteins and fatty substances secreted by the body, and in particular sebum. The invention thus makes it possible to eliminate the organic compounds as they appear, and to ensure continuous cleanliness of the keratin materials.

Furthermore, when the keratin materials are in contact with an object, a greater or lesser transfer of organic compounds from the surface of the keratin materials to the surface of the object often occurs. This may occur in particular for textiles in contact with the skin, the scalp, the hair, and generally for any object in contact with these. The invention also makes it possible to prevent and/or reduce the deposits at the surface of these objects.

The invention does not need to expose the keratin materials or surfaces treated to an intense light source, insofar as the oxides according to the invention remain effective, including in the presence of a weak or moderate source of light exposure, in particular natural light. Furthermore, the metal oxides according to the invention are effective, including in the absence of direct exposure to a light source, both in an outside environment (including at a low level of sunshine) and in an inside environment.

The present invention makes it possible to destroy the oily excretions of keratin materials such as in particular sebum, and the compounds resulting from the conversion of these excretions, such as for example aldehyde or ketone or peroxide derivatives resulting from oxidation by oxygen or ozone or microbial agents. Thus, the invention makes it possible to reduce the inconveniences caused by these oily excretions such as the shiny, staining appearance or the oily odours or the irritant compounds that may lead to reactions of discomfort such as desquamation. The present invention also makes it possible to destroy the organic compounds resulting from aqueous body secretions and the compounds resulting from the conversion of the organic compounds resulting from these aqueous excretions, such as for example the acid derivatives resulting from oxidation by oxygen or ozone or microbial agents, etc. Thus, the inconveniences of these aqueous excretions, such as the tacky, staining, clothes-marking or odorous aspect, are reduced.

The undesirable compounds may develop by secretion from the body, but may also be transported by the air or be deposited by contact on the keratin materials. The present invention makes it possible to reduce the amount thereof, and thus enables the user to overcome the drawbacks of pollution, odours, bacteria, germs and other microbes capable of accumulating on the keratin materials.

According to one variant, the oxide particles according to the invention are used to eliminate or reduce the amount of compounds which are deposited on the objects that come into contact with the keratin materials. This is particularly the case for fatty substances such as those resulting from contacts with the body during the handling of objects, and which give the objects a stained appearance. This is also the case for volatile organic molecules such as fragrances, or odours from humans and animals, compounds which, after deposition, evolve to give malodorous compounds, and give a malodorous aspect to the objects. To combat these inconveniences, the oxide particles according to the invention may be applied to or integrated into the surface of the objects.

The use according to the invention makes it possible in particular to eliminate the fatty substances from the surface of the keratin materials or objects in contact therewith. It makes it possible to reduce the oiling phenomena of the keratin materials, and in particular the natural re-oiling thereof due to secretions of sebum.

The invention also relates to a method for the cosmetic treatment of keratin materials or of the surface of an object in contact with the keratin materials, comprising:
(1) applying to said materials or to said surface a cosmetic composition comprising solid particles comprising at least one metal oxide OM1 of formula AₐB_{b}Oₙ₋ₓ as defined above; then
(2) exposing said materials or said surface to natural or artificial light.

According to a first preferred embodiment, the particles comprising the metal oxide OM1 are prepared by a flame spray pyrolysis (FSP) method.

According to a second embodiment that is also preferred, the metal oxide OM1 is used in combination with a second metal oxide OM2, different from the oxide OM1, and as described below, it being possible for said second oxide OM2 to be present in the particles comprising the oxide OM1, or in different solid particles.

Other subjects, characteristics, aspects and advantages of the invention will become even more clearly apparent on reading the description and the example which follows.

In the present description, and unless otherwise indicated:
- the expression "at least one" is equivalent to the expression "one or more" and can be replaced therewith;
- the expression "between" is equivalent to the expression "ranging from" and can be replaced therewith, and implies that the limits are included;
- the expression "human keratin materials" includes in particular the skin, scalp, nails and also human keratin fibres such as the hair, body hair, eyelashes and eyebrows;
- an "alkyl" is understood to mean an "alkyl radical", i.e. a C₁ to C₁₀, particularly C₁ to C₈, more particularly C₁ to C₆, and preferentially C₁ to C₄, linear or branched hydrocarbon-based radical, such as methyl, ethyl, propyl, isopropyl, n-butyl, isobutyl or tert-butyl;
- an "aryl" radical is understood to mean a monocyclic or fused or non-fused polycyclic carbon-based group, comprising from 6 to 22 carbon atoms, at least one ring of which is aromatic; preferentially, the aryl radical is a phenyl, biphenyl, naphthyl, indenyl, anthracenyl or tetrahydronaphthyl, preferably a phenyl;
- an "arylate" radical is understood to mean an aryl group which comprises one or more -C(O)O⁻ carboxylate groups, such as naphthalate or naphthenate;
- "complexed metal" is understood to mean that the metal forms a "metal complex" or "coordination compounds" in which the metal ion, corresponding to the central atom, is chemically bonded to one or more electron donors (ligands);
- a "ligand" is understood to mean a coordinating organic chemical group or compound, i.e. which comprises at least one carbon atom and which is capable of coordinating with a metal, and which, once coordinated or complexed, results in metal compounds corresponding to principles of a coordination sphere with a predetermined number of electrons (internal complexes or chelates) - see Ullmann's Encyclopedia of Industrial Chemistry, "Metal complex dyes", 2005, pp. 1-42. More particularly, the ligand(s) are organic groups which comprise at least one group that is electron-donating via an inductive and/or mesomeric effect, more particularly bearing at least one amino, phosphino, hydroxy or thiol electron-donating group, or the ligand is a persistent carbene, particularly of "Arduengo" type (imidazol-2-ylidenes) or comprises at least one carbonyl group. As ligand, mention may more particularly be made of: i) those which contain at least one phosphorus atom -P< i.e. phosphines such as triphenyl phosphines; ii) bidendate ligands of formula R-C(X)-CR'R"-C(X)-R‴ with R and R"", which are identical or different, representing a linear or branched (C₁-C₆)alkyl group, and R' and R", which are identical or different, representing a hydrogen atom or a linear or branched (C₁-C₆)alkyl group, preferentially R' and R" represent a hydrogen atom, X represents an oxygen or sulfur atom, or an N(R) group with R representing a hydrogen atom or a linear or branched (C₁-C₆)alkyl group, such as acetylacetone or β-diketones; iii) (poly)hydroxy carboxylic acid ligands of formula [HO-C(O)]n-A-C(O)-OH and the deprotonated forms thereof with A representing a monovalent group when n has the value zero or a polyvalent group when n is greater than or equal to 1, which is saturated or unsaturated, cyclic or non-cyclic and aromatic or non-aromatic based on a hydrocarbon comprising from 1 to 20 carbon atoms which is optionally interrupted by one or more heteroatoms and/or is optionally substituuted, notably with one or more hydroxyl groups; preferably, A represents a monovalent (C₁-C₆)alkyl group or a polyvalent (C₁-C₆)alkylene group optionally substituted with one or more hydroxyl groups; and n representing an integer between 0 and 10 inclusive; preferably, n is between 0 and 5, for instance among 0, 1 or 2; such as lactic, glycolic, tartaric, citric and maleic acids, and arylates such as naphthalates; and iv) C₂ to C₁₀ polyol ligands, comprising from 2 to 5 hydroxyl groups, notably ethylene glycol, glycerol, more particularly still the ligand(s) bear a carboxy, carboxylate or amino group, particularly the ligand is chosen from acetate, (C₁-C₆)alkoxylate, (di)(C₁-C₆)alkylamino, and arylate, such as naphthalate or naphthenate, groups;

The term "fuel" is understood to mean a liquid compound which, with dioxygen and energy, is burnt in a chemical reaction generating heat: combustion. In particular the liquid fuels are chosen from protic solvents, in particular alcohols such as methanol, ethanol, ispropanol, n-butanol, glycol; aprotic solvents in particular chosen from esters such as methyl esters and those derived from acetate, such as 2-ethylhexyl acetate, acids such as acetic acid, 2-ethylhexanoic acid (EHA), acyclic ethers such as ethyl ether, methyl tert-butyl ether (MTBE), methyl tert-amyl ether (TAME), methyl tert-hexyl ether (THEME), ethyl tert-butyl ether (ETBE), ethyl tert-amyl ether (TAEE), diisopropyl ether (DIPE), cyclic ethers such as tetrahydrofuran (THF), aromatic hydrocarbons or arenes such as xylene, non-aromatic hydrocarbons; and mixtures thereof. The fuels may optionally be chosen from liquefied hydrocarbons such as acetylene, methane, propane or butane; and mixtures thereof.

### The first metal oxide OM1

The solid particles used in the present invention contain at least one metal oxide OM1 of formula AₐB_{b}Oₙ₋ₓ, in which::
A denotes a metal chosen from bismuth, calcium, sodium, lanthanum, barium, copper, tin, magnesium, zinc and silver;
B denotes a metal chosen from tungsten, vanadium, gallium, niobium and strontium;
a is an integer within the range of from 1 to 4;
b is an integer within the range of from 1 to 10;
n is an integer within the range of from 3 to 6; and
x is a decimal number of less than n, within the range of from 0.1 to 5.
A is preferably chosen from bismuth and barium, and more preferably A denotes bismuth.
B is preferably chosen from tungsten and vanadium, and more preferably B denotes tungsten.

The integers a and b and x depend on the nature of the metals A and B.

The decimal number x is preferably within the range of from 0.1 to 1.9, more preferentially from 0.2 to 1.5, and better still from 0.3 to 1. Particularly preferably, x is greater than or equal to 0.5 and strictly less than 1.

According to one particular preferred embodiment, the oxide OM1 has the formula Bi₂WO₆₋ₓ, with x a decimal number within the range of from 0.2 to 1,5, preferably from 0.3 to 1; and better still x is greater than or equal to 0.5 and strictly less than 1.

Preferably, the metal oxide OM1 is in the crystalline state.

### The optional second metal oxide OM2

According to one preferred embodiment, the metal oxide OM1 is combined with a second metal oxide denoted OM2, which corresponds to the formula M_{c}Oₖ, in which:
M denotes a transition metal;
c is an integer within the range of from 1 to 3; and
k is a decimal number within the range of from 0.1 to 4.

The combined use of the two types of oxides OM1 and OM2 makes it possible to further increase the kinetics of photoconversion of the undesirable organic compounds. This makes it possible to increase the effectiveness of the elimination of these compounds, provided there is identical light exposure, or to retain a good effectiveness including in the presence of light exposure of low intensity, for example during use inside, or in winter.

Preferably, M denotes copper.

According to one particularly preferred embodiment, the oxide OM2 has the formula CuO.

Preferably, the metal oxide OM2 is in the crystalline state.

The metal oxide OM2 may be either present in the particles comprising the metal oxide OM1 (denoted particles P1), or in the form of different particles P2, as described below.

Preferably, the metal oxide OM2 is used in an amount smaller than that of the metal oxide OM1. More preferentially, the molar ratio of the amount of metal oxide OM1 to the amount of metal oxide OM2 is greater than or equal to 1, preferably greater than or equal to 2, more preferentially greater than or equal to 3, even more preferentially greater than or equal to 4, and better still greater than 4..

### The solid particles of metal oxides

In that which follows, P1 denotes the particles comprising the metal oxide OM1.

Said particles P1 comprise the metal oxide OM1, alone or combined with another metal oxide, such as in particular the oxide OM2 described above.

According to one preferred embodiment, the particles P1 do not comprise an upper coating layer.

According to one particularly preferred embodiment, the particles P1 are entirely constituted of said oxide OM1.

The particles P1 preferably have a number-average diameter ranging from 1 to 1000 nm, more preferably from 10 to 200 nm.

According to one embodiment that is also preferred, said second oxide OM2 is in the form of solid particles P2, different from said particles P1, and which preferably have a number-average diameter ranging from 1 to 300 nm, preferably from 2 to 20 nm, and more preferentially still from 2 to 10 nm.

In the latter embodiment, preferably the number-average diameter of the particles P1 is greater than that of said particles P2, and more preferentially the number-average diameter of the particles P1 is greater than or equal to twice that of the particles P2.

In the latter embodiment, preferably the particles P2 containing the oxide OM2 are joined to the particles P1 containing the oxide OM1. The particles may in particular be in the form of clusters in which either the particles P1 are joined to one another and/or to the particles P2 (each particle P1 may either be in contact with particles P1 and particles P2) or each of the particles P1 is joined only to particles P2 (the particles P2 are inserted between the particles P1 so that the particles P1 are not in direct contact with one another).

The number-average diameter of the particles P1 and P2 according to the invention may be determined by transmission electron microscopy or by X-ray diffraction (XRD).

### Preparation of the solid particles

According to one preferred embodiment, the solid particles P1 comprising said at least one metal oxide OM1 used in the present invention are obtained, or capable of being obtained, by flame spray pyrolysis (FSP).

Similarly, when particles P2 comprising a metal oxide OM2 are used, these particles P2 are preferably obtained, or capable of being obtained, by flame spray pyrolysis.

According to one preferred embodiment, the oxides OM1 and OM2 are prepared in a single FSP device, in the same flame.

Flame spray pyrolysis or FSP is a well-known method, which has essentially been developed for the synthesis of ultrafine powders of single or mixed oxides of various metals (for example SiO₂, Al₂O₃, B₂O₃, ZrO₂, GeO₂, WO₃, Nb₂O₅, SnO₂, MgO, ZnO), with controlled morphologies, and/or the deposition thereof on various substrates, by starting from a wide variety of metal precursors, generally in the form of organic or inorganic, preferably inflammable, sprayable liquids; the liquids sprayed into the flame, by being burnt, notably emit nanoparticles of metal oxides which are sprayed by the flame itself onto these various substrates. This method has also been used to manufacture oxide particles covered with a layer of silica. The principle of this method has been recalled for example in the recent (2011) publication by Johnson Matthey entitled "Flame Spray Pyrolysis: a Unique Facility for the Production of Nanopowders", Platinum Metals Rev., 2011, 55, (2), 149-151. Numerous variants of FSP processes and reactors have also been described, by way of example, in the patents or patent applications: US 5 958 361, US 2 268 337, WO 01/36332 or US 6 887 566, WO 2004/005184 or US 7 211 236, WO 2004/056927, WO 2005/103900, WO 2007/028267 or US 8 182 573, WO 2008/049954 or US 8 231 369, WO 2008/019905, US 2009/0123357, US 2009/0126604, US 2010/0055340, WO 2011/020204.

According to one preferred embodiment, the solid particles P1 comprising said at least one metal oxide OM1 are prepared by a process comprising at least the following steps:
a. preparing a composition (C) by adding one or more metal A precursors and one or more metal B precursors to a combustible solvent or to a mixture of combustible solvents; then
b. in a flame spray pyrolysis device, forming a flame by injecting the composition (C) and an oxygen-containing gas until particles P1 of metal oxide OM1 of formula AₐB_{b}Oₙ₋ₓ as defined above are obtained.

The precursors of the metals A and B and the combustible solvents that can be used according to the invention may be chosen from the metal precursors and the combustible solvents conventionally used in flame spray pyrolysis.

Preferably, the metal A precursor and the metal B precursor included in the composition (C) respectively comprise one or more atoms of A and B optionally complexed with one or more ligands containing at least one carbon atom.

More preferentially, said ligand(s) are chosen from acetate, nitrate, ammonium, (C₁-C₆)alkoxylate, (di)(C₁-C₆)alkylamino, and arylate, such as naphthalate or naphthenate, groups.

Preferably, the combustible solvent(s) are chosen from protic combustible solvents, aprotic combustible solvents, and mixtures thereof; more preferentially from alcohols, esters, acids, acyclic ethers, cyclic ethers, aromatic hydrocarbons or arenes, non-aromatic hydrocarbons, and mixtures thereof; and better still from (Ci-C₈)alkylcarboxylic acids such as acetic acid or 2-ethylhexanoic acid (EHA), (C₁-C₆)alkane(mono/di/tri)ols such as ethylene glycol, (Ci-Cs)alkylcarbonyl(Ci-C₈)alkoxys such as 2-ethylhexyl acetate, di(C₁-C₆)alkyl ethers such as ethyl ether, methyl tert-butyl ether (MTBE), methyl tert-amyl ether (TAME), methyl tert-hexyl ether (THEME), ethyl tert-butyl ether (ETBE), ethyl tert-amyl ether (TAEE), diisopropyl ether (DIPE), tetrahydrofuran (THF), xylene, and mixtures thereof.

According to one preferred embodiment, the combustible solvent(s) are chosen from aprotic combustible solvents comprising at least three carbon atoms and mixtures thereof; more preferentially still from xylene, tetrahydrofuran, 2-ethylhexyl acetate, and mixtures thereof.

According to another embodiment, the combustible solvent(s) are chosen from protic combustible solvents comprising at least two carbon atoms and mixtures thereof; more preferentially still from acetic acid, ethylene glycol, 2-ethylhexanoic acid (EHA), and mixtures thereof.

Advantageously, the total content of precursor of metals A and B in the composition (C) is between 1% and 90% by weight, in particular between 5% and 80% by weight, preferably between 10% and 70% by weight, more preferentially between 25% and 60% by weight, relative to the total weight of composition (C).

The process for preparing the particles P1 further comprises a step (b) of injecting composition (C) and an oxygen-containing gas into a flame spray pyrolysis (FSP) device to form a flame.

During this step (b), composition (C) and the oxygen-containing gas are advantageously injected into the flame spray pyrolysis device, by two injections that are separate from one another. In other words, composition (C) and the oxygen-containing gas are injected separately, i.e. composition (C) and the oxygen-containing gas are not injected by means of a single nozzle.

More particularly, composition (C) is transported by one tube, whereas the oxygen-containing gas (also referred to as "dispersion Oxygen") is transported by another tube. The inlets of the two tubes are arranged so that the oxygen-containing gas produces a negative pressure and, via a Venturi effect, causes the composition (C) to be sucked up and converted into droplets.

Step (b) may optionally further comprise an additional injection of a "premix" mixture comprising oxygen and one or more combustible gases. This "premix" mixture is also referred to as "supporting flame oxygen" and enables the production of a support flame intended to ignite and maintain the flame resulting from composition (C) and the oxygen-containing gas (i.e. "dispersion Oxygen").

Preferably, during step (b), composition (C), the oxygen-containing gas and optionally the "premix" mixture when it is present, are injected into a reaction tube, also referred to as an "enclosing tube". Preferably, this reaction tube is made of metal or of quartz. Advantageously, the reaction tube has a height of greater than or equal to 20 cm, more preferentially greater than or equal to 40 cm, and better still greater than or equal to 50 cm. Advantageously, the length of said reaction tube is between 30 cm and 300 cm, preferably between 40 cm and 200 cm, more preferentially between 45 cm and 100 cm, and better still this length is equal to 50 cm.

The weight ratio of the mass of solvent(s) present in composition (C) on the one hand, to the mass of oxygen-containing gas on the other hand, is defined as follows:
Firstly, the amount of oxygen-containing gas (also referred to as oxidizer compound) is calculated in order for the assembly formed by composition (C), i.e. the combustible solvent(s) and the metal precursor(s) on the one hand, and the oxygen-containing gas on the other hand, to be able to react together in a combustion reaction in a stoichiometric ratio (therefore without an excess or deficit of oxidizer compound).
Starting from this calculated amount of oxygen-containing gas, also referred to as "calculated oxidizer", a new calculation is performed to deduce therefrom the amount of oxygen-containing gas to be injected, also referred to as "oxidizer to be injected", according to the formula: Oxidizer to be injected = Calculated oxidizer / ϕ
with ϕ preferably between 0.30 and 1.0, and more preferentially between 0.7 and 1.0.

This method is notably defined by Turns, S. R. in An Introduction to Combustion: Concepts and Applications, 3rd ed.; McGraw-Hill: New York, 2012.

Preferably, the flame formed during step (b) is at a temperature above or equal to 2000°C, in at least one part of the flame.

The solid particles P2 comprising a metal oxide OM2 may be prepared by following the same principle as the one described above for the preparation of the particles P1.

According to one preferred embodiment, the solid particles P2 comprising said at least one metal oxide OM2 are prepared by a process comprising at least the following steps:
a. preparing a composition (C') by adding one or more metal M precursors to a combustible solvent or to a mixture of combustible solvents; then
b. in a flame spray pyrolysis device, forming a flame by injecting the composition (C') and an oxygen-containing gas until particles P2 of metal oxide OM2 of formula M_{c}Oₖ as defined above are obtained.

A person skilled in the art will easily know how to adapt the detailed description described above for the preparation of the particles P1 to the preparation of the particles P2.

According to one preferred embodiment, the solid particles P1 and P2 are prepared simultaneously, in the same flame spray pyrolysis device.

In this embodiment, the solid particles P1 comprising said at least one metal oxide OM1 are prepared jointly with the solid particles P2 comprising said at least one metal oxide OM2 by a process comprising at least the following steps:
a. preparing a first composition (C) on the one hand by adding one or more metal A precursors, and one or more metal B precursors to a combustible solvent or to a mixture of combustible solvents, and a second composition (C'), on the other hand by adding one or more metal M precursors to a combustible solvent or to a mixture of combustible solvents; then
b. in a flame spray pyrolysis device, forming a flame by injecting the compositions (C) and (C') and an oxygen-containing gas until particles P1 of metal oxide OM1 of formula AₐB_{b}Oₙ₋ₓ as defined above and particles P2 of metal oxide OM2 of formula M_{c}Oₖ as defined above are obtained.

According to one preferred embodiment, use is made of a single composition (C") containing one or more metal A precursors, one or more metal B precursors and one or more metal M precursors in a combustible solvent or in a mixture of combustible solvents, instead of two separate compositions (C) and (C').

### Use

The use according to the invention of said solid particles P1 comprising at least one metal oxide OM1 aims to reduce, or even eliminate, the deposits of undesirable organic compounds at the surface of keratin materials, in particular human keratin materials, or at the surface of objects that come into contact with said materials.

Preferably, said keratin materials are chosen from the skin, scalp, and hair.

Preferably, the undesirable organic compounds are chosen from the proteins and fatty substances secreted by the body. The invention aims in particular to prevent, reduce and/or eliminate the deposits of sebum at the surface of the keratin materials, or at the surface of objects in contact with the keratin materials.

For the implementation of the invention, the particles P1 and, optionally, the particles P2 described above, are brought into contact with the keratin materials or the surfaces to be treated. The keratin materials or the surfaces to be treated are then exposed to light.

According to one embodiment of the invention, the particles P1 and, optionally, the particles P2 described above, are applied directly to the keratin materials or to the surfaces to be treated. Said particles may be applied as they are, in the form of a powder, or else in a form dispersed in a cosmetic composition.

According to another embodiment of the invention, the particles P1 and, optionally, the particles P2 described above, are integrated into an article intended to be applied to the keratin materials. The particles may be incorporated into said article in particular in the case of an absorbent article, or impregnated into the surface of the article in particular if the latter is not absorbent (such as an article made of leather for example).

The article may be of textile type, for example a piece of fabric which may be absorbent, such as a towel, a tea towel, absorbent paper such as kitchen roll, or non-absorbent.

The impregnation of the particles on the article may be carried out by sprinkling using a powder of said particles, optionally followed and/or preceded by a fixing step; or by application of a composition comprising said particles in dispersion in a solvent, which is then left to evaporate.

The article may be a brush, in particular for keratin fibres such as a hairbrush, said brush comprising a coating of particles according to the invention. Said brush is then applied to the keratin materials to be treated, for example in the case of the hair, it is combed one or more times with said brush. The brush is preferably then exposed to natural or artificial light. In particular, the brush can be exposed to a very strong light (> 0.1 W/cm²) in a chamber designed so that the light cannot escape therefrom. The treated keratin fibres may also then be treated again with said brush exposed to natural or artificial light, as many times as necessary. The brush is thus kept clean in the sense that the organic compounds that it absorbs during contact are eliminated during the light exposure. It is possible to wash the brush from time to time, typically once every 10 uses, by application of water or a washing composition.

According to one variant, the brush further comprises at least one means for generating artificial light. According to one particular embodiment, the particles are located at the base of the bristles of the brush and artificial light emitters are positioned at the other end of the bristles. After one or more passes of the brush over the keratin fibres, the sebum is rapidly destroyed

According to another variant of the invention, the particles P1 and, optionally, the particles P2 are used in a collector device. A collector is understood to mean a device that makes it possible to collect the organic compounds, such as sebum, after rubbing with the keratin materials. The collector device may be a system which does not require handling such as a cloth, a shell, a piece of clothing, or a hat. The collector device can then be left in natural or artificial light in order for the organic compounds collected to be eliminated.

### The treatment method

According to a first embodiment, the invention relates to a method for the cosmetic treatment of keratin materials or of the surface of an object in contact with the keratin materials, comprising:
(1) applying to said materials or to said surface a cosmetic composition comprising solid particles comprising at least one metal oxide OM1 of formula AₐB_{b}Oₙ₋ₓ as described above; then
(2) exposing said materials or said surface to natural or artificial light.

Preferably, the cosmetic composition used in step (1) further comprises at least one metal oxide OM2 of formula M_{c}Oₖ as described above, present in the particles P1 or in different solid particles P2 as described above.

Said cosmetic composition may be in various galenical forms. Thus, the composition of the invention may be in the form of a (pulverulent) powder comprising the particles P 1, and optionally the particles P2, alone or as a mixture with a pulverulent support.

The cosmetic composition may also be in the form of a more or less fluid liquid, for example in the form of a simple or complex emulsion (oil-in-water, or abbreviated to O/W, water-in-oil or W/O, oil-in-water-in-oil or O/W/O, or water-in-oil-in-water or W/O/W), such as a cream, a milk or a cream gel, or in the form of a gel, a paste or a composition for an aerosol device.

The cosmetic composition comprises the particles of metal oxide(s) according to the invention, preferably dispersed in a cosmetic medium. A "cosmetic medium" is understood to mean a medium suitable for application to human materials.

According to one preferred embodiment, said cosmetic medium comprises water and/or one or more organic solvents. Preferably, the composition comprises water, in a content notably of between 5% and 95% by weight relative to the total weight of the composition.

The term "organic solvent" means an organic substance that is capable of dissolving another substance without chemically modifying it. As examples of organic solvents that can be used in the composition of the invention, mention may for example be made of lower C₂-C₆ alkanols, such as ethanol and isopropanol; polyols and polyol ethers, for instance 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.

When they are present, the organic solvent(s) are present in proportions preferably between 0.1% and 40% by weight, more preferentially between 1% and 30% by weight and even more particularly between 5% and 25% by weight relative to the total weight of the composition.

The composition according to the invention may further contain a fatty phase and may be in the form of a direct or inverse emulsion.

According to one particular embodiment, the cosmetic composition may also be in the form of an anhydrous composition, for instance in the form of an oil or an alcoholic solution. The term "anhydrous composition" is intended to mean a composition containing less than 2% by weight of water, preferably less than 1% by weight of water, and even more preferentially less than 0.5% by weight of water relative to the total weight of the composition, or even a composition that is free of water. In compositions of this type, the water possibly present is not added during the preparation of the composition, but corresponds to the residual water provided by the mixed ingredients.

The composition according to the invention may be prepared according to the techniques that are well known to those skilled in the art.

The content of the metal oxide OM1 in the cosmetic composition ranges preferably from 0.4% to 40% by weight, more preferentially from 0.5% to 20% by weight, better still from 1% to 10% by weight and more preferentially still from 1.5% to 5% by weight, relative to the total weight of the composition.

When a metal oxide OM2 is also present, its content ranges preferably from 0.1% to 10% by weight, preferably from 0.15% to 5% by weight, and better still from 0.25% to 1.5% by weight, relative to the total weight of the composition.

According to one preferred embodiment, the cosmetic composition comprises the two metal oxides OM1 and OM2 described above, with a molar ratio of the content of metal oxide OM1 to the content of metal oxide OM2 of greater than or equal to 1, preferably greater than or equal to 2, more preferentially greater than or equal to 3, even more preferentially greater than or equal to 4, and better still greater than 4.

The composition may also contain one or more additives chosen from thickeners, fragrances, nacreous agents, preservatives, sunscreens, anionic, nonionic, amphoteric or cationic surfactants, anionic or nonionic or amphoteric polymers, cationic polymers, proteins, protein hydrolysates, ceramides, pseudoceramides, fatty acids with linear or branched C₁₆-C₄₀ chains such as 18-methyleicosanoic acid, hydroxy acids, vitamins, provitamins such as panthenol, silicones, plant oils, mineral oils and synthetic oils, antidandruff agents and any other additive conventionally used in the cosmetic field that does not adversely affect the stability and the properties of the compositions according to the invention.

These additives are optionally present in the composition according to the invention in proportions that can range from 0.001% to 50% by weight, relative to the total weight of the composition. The precise amount of each additive is readily determined by those skilled in the art depending on its nature and its function.

The application of the composition to the keratin materials or to the surfaces to be treated may be carried out with the hands, with a sprayer, with an aerosol, with an applicator end piece, with a dispenser comb or with a towel (or a wipe) impregnated with the composition.

This application may be followed by drying of the keratin materials or of the surfaces to be treated. The drying phase may optionally be carried out by heating using a hairdryer, a heating hood, a straightening or curling iron, a heating comb or by any other heating device.

After application of the composition described above to the keratin materials, or to the surfaces to be treated, these are exposed to light.

The term light denotes electromagnetic radiation, which may be of ultraviolet (UV) and/or visible and/or near infrared (NIR) type. It may be natural light (in the case in particular of use in an outside or inside environment, for example close to a window) or artificial light, in particular from a lamp emitting UV and/or visible radiation (for example in the case of use in an inside environment).

Preferably, step (2) of the cosmetic treatment method is carried out by photoirradiation, using a light source (or lamp) that emits one or more electromagnetic waves with a wavelength of between 200 nm in the ultraviolet (UV) range and 3000 nm in the infrared (IR) range.

The term "photoirradiation" is understood to mean any exposure of the keratin materials to an artificial light wave, i.e. emitted by a lamp. The light spectrum may comprise wavelengths within the UV region (200-400 nm), the visible region (400-750 nm) and the infrared region (745 nm to 3 µm).

For the lamps emitting in the UV region, mention may be made of those described in Ullmann's Encyclopedia "Ultraviolet and Visible Spectroscopy", 2008, Wiley-VCH Verlag GmbH & Co. KGaA, Weinheim, 10.1002/14356007.b05 383.pub2, point 3.2. For the lamps in general, mention may be made of those mentioned in Ullmann's Encyclopedia "Lamps" 2005, Wiley-VCH Verlag GmbH & Co. KGaA, Weinheim 10.1002/14356007.a15 115, and Ullmann's Encyclopedia "Photochemistry" 2005, Wiley-VCH Verlag GmbH & Co. KGaA, Weinheim 10.1002/14356007.a19 573, point 3.2 "*light sources*".

The lamps used in the method of the invention may in particular be incandescent, halogen or fluorescent lamps; low-pressure lamps, for example sodium or neon lamps, high-pressure lamps, for example mercury lamps, halide lamps, flash lamps, for example xenon flash lamps, fluorescent excimer lamps, such as xenon fluorescent excimer lamps, light emitting diodes or LEDs of 50 to 1000 mW, lamps emitting black light or Wood's light, and lasers.

According to one preferred embodiment, use is made, as light source, of an LED lamp emitting radiation with a wavelength in the visible region (400-745 nm).

According to one also preferred variant, step (2) of the cosmetic treatment method is carried out by exposing the keratin materials to natural sunlight or daylight.

The exposure to light of the keratin materials or of the surface to be treated is preferably carried out for a period ranging from 10 minutes to 180 minutes, more preferentially from 30 to 90 minutes.

According to a second embodiment, the invention relates to a method for the cosmetic treatment of keratin materials, comprising:
(1) bringing said materials into contact with an article comprising solid particles comprising at least one metal oxide OM1 of formula AₐB_{b}Oₙ₋ₓ as defined above; then
(2) exposing said article to natural or artificial light.

Preferably, the article used in step (1) further comprises at least one metal oxide OM2 of formula M_{c}Oₖ as described above, present in the particles P1 or in different solid particles P2 as described above.

The article used in step (1) may be any object capable of being applied to the keratin materials, and in particular a textile article or a brush, as described above. During step (1), the undesirable organic compounds are deposited by transfer on the surface of said article.

Step (2) is carried out by exposing said article to light. The conditions of such an exposure are those described above regarding the first embodiment. This light exposure enables the destruction of the undesirable organic compounds by photodegradation.

The following examples serve to illustrate the invention without, however, being limiting in nature.

### Examples

In the examples which follow, the particles are prepared in a flame spray pyrolysis device (or FSP device) in which the liquids are provided to the burner via a capillary nozzle (500 µm diameter capillary). The flame produced is enclosed in a tube surrounding the pyrolysis flame. The tube is positioned above the nozzle of the FSP device. The tube is a cylinder made of metal or quartz having a length of between 20 and 60 cm, in particular between 30 and 50 cm, for instance 40 cm. The flame generally has a length of between from 5 to 15 cm and is at a temperature of between 500°C and 2000°C. The precursors of metals (such as Bi, W, Cu) which are sprayed are burnt in the flames, leading to the formation of particles. The particles are then collected upstream on a glass fibre filter which is maintained preferably at a temperature of between 350°C and 420°C. The distance between the FSP nozzle and the filter for collecting the particles is between 40 and 80 cm, preferably between 55 and 65 cm.

### Example 1: Preparation of particle powders by FSP

A composition C1 is prepared by dissolving 250 mM of ammonium metatungstate hydrate and 500 mM of bismuth nitrate in an organic solvent constituted of a diethylene glycol monobutyl ether/absolute ethanol/acetic acid mixture.

Next, this composition C1 and also pure oxygen are injected into the FSP device.

### 1.1. Preparation of Bi₂WO₆₋ₓ particles (invention)

The flow rates used for the injection are 7 ml/min of composition C1 and 4 l/min of gas (O₂).

A powder is thus obtained, which is subjected to a thermal post-treatment by exposing it to a temperature of 300°C for 1 hour, which makes it possible to improve its crystallinity.

The powder has a yellow-green colour and contains Bi₂WO₆₋ₓ particles with x being a decimal number greater than 0.1 and strictly less than 1.

The number-average size of the particles is 25 nm (measured by X-ray diffraction or XRD).

### 1.2. Preparation of Bi₂WO₆ particles (comparative)

The flow rates used for the injection are 7 ml/min of composition C1 and 5 l/min of gas (O₂).

A powder is thus obtained, which is subjected to a thermal post-treatment by exposing it to a temperature of 300°C for 1 hour.

The powder has a yellow-green colour and contains Bi₂WO₆ particles.

The number-average size of the particles is 25 nm (measured by X-ray diffraction or XRD).

### 1.3. Characterization of the particles

The Bi₂WO₆₋ₓ and Bi₂WO₆ particles prepared in 1.1 and 1.2 above are differentiated by Raman spectroscopy in the visible zone between 400 and 500 nm in particular by a higher absorbance (typically around 20% higher) for the Bi₂WO₆ particles compared to those of Bi₂WO₆₋ₓ.

The two types of particles are also differentiated by electron paramagnetic resonance spectroscopy (or EPR spectroscopy). The EPR spectrum of the Bi₂WO₆₋ₓ particles prepared in 1.1 above typically has a signal at g = 2.002, which is characteristic of an oxygen atom vacancy in the crystal lattice of Bi₂WO₆. The method is very sensitive and ensures that there is indeed a suboxide present with x greater than 0.1.

On the contrary, the Bi₂WO₆₋ₓ and Bi₂WO₆ particles are not generally differentiated by the positioning of the peaks in XRD.

Generally, the metal oxide particles according to the invention are suboxides (for example of formula Bi₂WO₆₋ₓ):
1) having, in X-ray diffraction (XRD), a positioning of the peaks that is similar to that of its stoichiometrically oxidized homologues (for example Bi₂WO₆);
2) having a Raman spectroscopy profile such that their absorbance in the zone 400-500 nm is at least 5% and preferably at least 10% lower than that of its stoichiometrically oxidized homologues (for example Bi₂WO₆); and/or
3) having an EPR spectroscopy profile comprising a signal in the region of 2, in particular g = 2.002, that its stoichiometrically oxidized homologues (in particular Bi₂WO₆) do not have.

### Example 2: Preparation of particle mixtures by FSP

A composition C2 is prepared by dissolving together 25 mM of copper nitrate, 250 mM of ammonium metatungstate hydrate and 500 mM of bismuth nitrate in an organic solvent constituted of a diethylene glycol monobutyl ether/absolute ethanol/acetic acid mixture.

Next, this composition C2 and also pure oxygen are injected into the FSP device.

### 2.1. Preparation of a mixture of Bi₂WO₆₋ₓ particles and CuO particles invention

The flow rates used for the injection are 7 ml/min of composition C2 and 4 l/min of gas (O₂).

A powder is thus obtained, which is subjected to a thermal post-treatment by exposing it to a temperature of 300°C for 1 hour. The powder has a light brown colour and is formed of a mixture of Bi₂WO₆₋ₓ particles (with x being a decimal number greater than 0.1 and strictly less than 1) and CuO particles.

The EPR spectrum of the powder obtained has a signal at g = 2.002, which is characteristic of an oxygen atom vacancy compared to the crystal lattice of Bi₂WO₆.

The number-average size of the particles is 20 nm (measured by XRD) for the Bi₂WO₆₋ₓ particles and 2.5 nm (measured by XRD) for the CuO particles.

The molar ratio of the amount of Bi₂WO₆₋ₓ relative to the amount of CuO in the powder is 20.

### 2.2. Preparation of a mixture of Bi₂WO₆ particles and CuO particles (comparative)

The flow rates used for the injection are 7 ml/min of composition C2 and 7 l/min of gas (O₂).

A powder is thus obtained, which is subjected to a thermal post-treatment by exposing it to a temperature of 300°C for 1 hour.

The powder has a light brown colour and is formed of a mixture of Bi₂WO₆ particles and CuO particles.

The number-average size of the particles is 20 nm (measured by XRD) for the Bi₂WO₆ particles and 2.5 nm (measured by XRD) for the CuO particles.

The molar ratio of the amount of Bi₂WO₆ relative to the amount of CuO in the powder is 20.

### Example 3: Tests for elimination of sebum in a thin layer under natural light irradiation

5 identical plates are prepared, by each time spreading a layer of artificial sebum (33 mg) composed of oleic acid on a glass plate having a surface area of around 10 cm².

Next, deposited on the surface of each plate, are 10 mg of one of the following powders:
On plate 1 and plate 5: the powder formed of the Bi₂WO₆₋ₓ particles from example 1.1 (invention) is deposited;
On plate 2: the powder formed of the Bi₂WO₆ particles from example 1.2 (comparative) is deposited;
On plate 3: the powder formed of the mixture of Bi₂WO₆₋ₓ and CuO particles from example 2.1 (invention) is deposited;
On plate 4: the powder formed of the mixture of Bi₂WO₆ and CuO particles from example 2.2 (comparative) is deposited.

Next, plates 1 to 4 are exposed to moderate daylight (38 mW/cm²) for a total duration of 60 minutes. Then, the plates are weighed to deduce the amount of sebum which has disappeared therefrom after 30 minutes, then after 60 minutes of light exposure.

The results obtained, in terms of percentage by weight of sebum that has disappeared, are given in detail in Table 1 below.

**[Table 1]**

| Plate | Degree of disappearance of the sebum at t = 30 min | Degree of disappearance of the sebum at t = 60 min |
|---|---|---|
| 1 - invention | 50% | 61% |
| 2 - comparative | 30% | 42% |
| 3 - invention | 82% | 93% |
| 4 - comparative | 31% | 39% |

In order to carry out a control, plate 5 (identical to plate 1) was not exposed to light. No disappearance of the sebum at t = 60 min was observed (degree of disappearance measured less than 5% by weight).

The results above show that the use of the particles according to the present invention makes it possible to eliminate the sebum significantly more effectively than the use of the comparative particles. The powder formed from the Bi₂WO₆₋ₓ particles gives excellent results, and the powder formed from the mixture of Bi₂WO₆₋ₓ and CuO particles makes it possible to further increase the effectiveness.

### Example 4: Tests for elimination of sebum on heads of hair

A head of hair comprising 1000 mg of sebum is considered. Half is on the area of the head of hair that is visible, namely a surface area of 1000 cm². Thus, on the visible surface area, there is 0.5 mg of sebum per cm². This amount is sufficient to give the hair a partially dirty appearance. In particular, when a comb is passed through the head of hair, the hairs have a tendency to remain clumped together revealing furrows at the location where the teeth of the comb have passed through.

### Test 1:

In a first test, a composition is produced by dispersing 1000 mg of the powder formed from the Bi₂WO₆₋ₓparticles from example 1.1 in 50 ml of ethanol. 12 grams of this composition are applied to the head of hair, then there is a waiting time of 10 minutes for the hair to dry.

The hair is then subjected to natural lighting (sunlight) in an inside environment (behind a window) estimated at 20 mW/cm². It is observed that 1 hour is needed to sufficiently eliminate the sebum that is on the surface and to reach a level where it becomes invisible.

In particular, it is observed that the hair treated is shiny with significantly better strand separation after combing than before treatment with combing. The treated hair appears clean as if it had just been washed, grease no longer appears on the hair, unlike the hair before treatment, which appears very greasy due to the presence of sebum.

### Test 2:

Test 1 is repeated, using a composition obtained by dispersing 1000 mg of the powder formed from the mixture of Bi₂WO₆₋ₓ and CuO particles from example 2.1 in 50 ml of ethanol.

It is observed that 28 minutes are needed to sufficiently eliminate the sebum that is on the surface and to reach a level where it becomes invisible.

### Test 3:

Test 1 is repeated, using 12 grams of ethanol (with no particles).

No change in the appearance of the head of hair is observed, the hair still appears greasy.

### Test 4:

Test 1 is repeated, using a composition obtained by dispersing 1000 mg of the powder formed from the mixture of Bi₂WO₆ and CuO particles from example 2.2 in 50 ml of ethanol.

After one hour, the hair is less greasy but does not appear clean. This is due to the fact that the elimination efficiency is insufficient, leaving around 35% to 40% of sebum on the visible surface of the head of hair.

### Test 5:

Test 2 is repeated, but leaving the hair in the dark after the 10 minutes of drying.

No change in the appearance of the head of hair is observed, the hair still appears greasy.

### Example 5: Test for maintaining the cleanliness of a head of hair

A cosmetic composition is produced by dispersing 400 mg of the powder formed from the Bi₂WO₆₋ₓ particles from example 1.1 in 50 ml of ethanol.

The test is carried out on a model having a head of mid-length hair which has just been washed in the evening at 22:00. The head of hair is sufficiently stripped of sebum for the hair to appear clean. Once the hair is dry, 12 grams of the cosmetic composition above is applied, targeting the roots and carrying out a massage at the roots with the fingertips. Overnight the model's scalp produces approximately 500 mg of sebum, which is distributed as the hours go by following various contacts (pillow). Then it produces another 500 mg of sebum during the day, here considered as ranging from 10:00 to 22:00. During this second period, the sebum is distributed also along the length of the hair following various contacts (hand, comb, etc.).

The illumination of the model's head of hair is started in the morning at a relatively low level of 10 mW/cm². From 08:00 to 09:00, owing to the application carried out the evening before of the composition comprising particles according to the invention, the 500 mg of sebum produced during the night are destroyed in 1 hour.

The rest of the day, the particles according to the invention continue to destroy the sebum that appears on the scalp then migrates to the roots before being taken away by contacts and other movements. At the end of the day, it is observed that the hair has remained generally clean. Thus, the model does not feel the need to wash their hair in the evening.

The next day, the hair is slightly dirty on waking, but regains a clean appearance during the morning in the presence of light.

### Example 6: Tests using a brush

A brush is produced by adhesive bonding, to a metal base, a series of 80 bristles made of absorbent foam that are 3 mm wide and 1 cm long. Then 12 ml of the composition from Example 4 - Test 2 (dispersion of 1000 mg of powder formed from the mixture of the Bi₂WO₆₋ₓ and CuO particles in 50 ml of ethanol) are sprayed onto the brush, which is then left to dry.

This brush, when it is passed through the hair and the roots, absorbs a portion of the sebum while retaining the particles at the surface of the foam. This property stems from the fact that the contact is dry (no solvent). Next, the brush is placed (bristles downwards) 5 cm above a light source (series of four 1 W LED lamps), until the sebum is eliminated.

One variant consists in using a base made of plexiglass and a series of diodes placed under the base. Thus, it is possible to brush the hair, collect the sebum and begin to eliminate the sebum from the moment of contact. Afterwards, when the user puts the brush down, the illumination continues in order to finish eliminating the sebum collected by the foam bristles.

### Example 7: Tests on packaging

For these tests, use is made of two identical copies of packaging made of brown cardstock intended to cover a box containing a cosmetic product such as a perfume.

In a first test, a tester places their fingers on this packaging, a visible mark remains on the packaging, owing to the fact that the tester's fingers are covered with a thin layer of sebum.

In the second test, the packaging is pretreated by spraying onto the cardstock an ethanolic dispersion of particles according to the invention. For this, 10 mg of the powder formed of the mixture of Bi₂WO₆₋ₓ and CuO particles (example 2.1) are dispersed in 10 ml of absolute ethanol, then this dispersion is sprayed onto the cardstock which is then left to dry. It is observed that when a tester places their fingers on this packaging, a visible mark remains on the packaging, owing to the fact that the fingers are covered with a thin layer of sebum.

Then the two packagings are arranged under light obtained from a 1 W yellow spotlight at 20 cm, representative of shop lighting.

After 10 minutes, it is observed that the finger marks on the packaging of the second test gradually disappear until they become invisible after 1 hour. This disappearance is not observed on the packaging from the first test.

## Claims

1. Use, for preventing, reducing and/or eliminating the deposits of organic compounds at the surface of keratin materials, in particular human keratin materials, or at the surface of objects in contact with said keratin materials, of solid particles comprising at least one metal oxide OM1 of formula AₐB_{b}Oₙ₋ₓ, in which:
A denotes a metal chosen from bismuth, calcium, sodium, lanthanum, barium, copper, tin, magnesium, zinc and silver;
B denotes a metal chosen from tungsten, vanadium, gallium, niobium and strontium;
a is an integer within the range of from 1 to 4;
b is an integer within the range of from 1 to 10;
n is an integer within the range of from 3 to 6; and
x is a decimal number of less than n, within the range of from 0.1 to 5.

2. Use according to claim 1, in which A is chosen from bismuth and barium, and preferably A denotes bismuth.

3. Use according to either one of the preceding claims, in which B is chosen from tungsten and vanadium, and preferably B denotes tungsten.

4. Use according to any one of the preceding claims, in which the oxide OM1 has the formula Bi₂WO₆₋ₓ, with x a decimal number within the range of from 0.2 to 1,5, preferably from 0.3 to 1; and better still x is greater than or equal to 0.5 and strictly less than 1.

5. Use according to any one of the preceding claims, in which the metal oxide OM1 is combined with a second metal oxide OM2 of formula M_{c}Oₖ, in which:
M denotes a transition metal;
c is an integer within the range of from 1 to 3; and
k is a decimal number within the range of from 0.1 to 4,
and preferably OM2 has the formula CuO.

6. Use according to claim 5, in which the molar ratio of the amount of metal oxide OM1 to the amount of metal oxide OM2 is greater than or equal to 1, preferably greater than or equal to 2, more preferentially greater than or equal to 3, even more preferentially greater than or equal to 4, and better still greater than 4.

7. Use according to any one of the preceding claims, in which the solid particles comprising said at least one metal oxide OM1 are obtained, or capable of being obtained, by flame spray pyrolysis.

8. Use according to any one of the preceding claims, for preventing, reducing and/or eliminating the deposits of proteins and fatty substances secreted by the body, and in particular sebum deposits.

9. Method for the cosmetic treatment of keratin materials or of the surface of an object in contact with the keratin materials, comprising:
(1) applying to said materials or to said surface a cosmetic composition comprising solid particles comprising at least one metal oxide OM1 of formula AₐB_{b}Oₙ₋ₓ as defined in any one of claims 1 to 4 and 7; then
(2) exposing said materials or said surface to natural or artificial light.

10. Method according to claim 9, **characterized in that** the cosmetic composition used in step (1) further comprises at least one metal oxide OM2 of formula M_{c}Oₖ as defined in any one of Claims 5 and 6, present in said particles comprising at least one metal oxide OM1 or in different solid particles.

11. Method according to either one of claims 9 and 10, **characterized in that** the content of metal oxide OM1 in the cosmetic composition ranges from 0.4% to 40% by weight, preferably from 0.5% to 20% by weight, better still from 1% to 10% by weight, and even more preferentially from 1.5% to 5% by weight, relative to the total weight of the composition.

12. Method according to any one of claims 9 to 11, **characterized in that** the content of metal oxide OM2 ranges from 0.1% to 10% by weight, preferably from 0.15% to 5% by weight and better still from 0.25% to 1.5% by weight, relative to the total weight of the composition.

13. Method for the cosmetic treatment of keratin materials, comprising:
(1) bringing said materials into contact with an article comprising solid particles comprising at least one metal oxide OM1 of formula AₐB_{b}Oₙ₋ₓ as defined in any one of claims 1 to 4 and 7; then
(2) exposing said article to natural or artificial light.

14. Method according to claim 13, **characterized in that** the article used in step (1) further comprises at least one metal oxide OM2 of formula M_{c}Oₖ as defined in any one of Claims Sand 6, present in said particles comprising at least one metal oxide OM1 or in different solid particles.

15. Method according to any one of claims 9 to 14, **characterized in that** step (2) is carried out by photoirradiation, using a light source that emits one or more electromagnetic waves with a wavelength of between 200 nm in the ultraviolet range and 3000 nm in the infrared range, and preferably using a LED lamp emitting radiation with a wavelength in the visible range.

## Patentansprüche

1. Verwendung von festen Teilchen, die mindestens ein Metalloxid OM1 der Formel AₐB_{b}Oₙ₋ₓ umfassen, zur Verhinderung, Verringerung und/oder Beseitigung der Ablagerungen von organischen Verbindungen auf der Oberfläche von Keratinmaterialien, insbesondere menschlichen Keratinmaterialien, oder an der Oberfläche von Gegenständen in Kontakt mit den Keratinmaterialien, wobei:
A für ein Metall steht, das aus Bismut, Calcium, Natrium, Lanthan, Barium, Kupfer, Zinn, Magnesium, Zink und Silber ausgewählt ist;
B für ein Metall steht, das aus Wolfram, Vanadium, Gallium, Niob und Strontium ausgewählt ist;
a eine ganze Zahl im Bereich von 1 bis 4 ist;
b eine ganze Zahl im Bereich von 1 bis 10 ist;
n eine ganze Zahl im Bereich von 3 bis 6 ist; und
x eine Dezimalzahl mit einem kleineren Wert als n im Bereich von 0,1 bis 5 ist.

2. Verwendung nach Anspruch 1, wobei A aus Bismut und Barium ausgewählt ist und A vorzugsweise für Bismut steht.

3. Verwendung nach einem der vorhergehenden Ansprüche, wobei B aus Wolfram und Vanadium ausgewählt ist und B vorzugsweise für Wolfram steht.

4. Verwendung nach einem der vorhergehenden Ansprüche, wobei das Oxid OM1 die Formel Bi₂WO₆₋ₓ aufweist, wobei x eine Dezimalzahl im Bereich von 0,2 bis 1,5, vorzugsweise von 0,3 bis 1, ist; und x noch besser größer oder gleich 0,5 und streng kleiner als 1 ist.

5. Verwendung nach einem der vorhergehenden Ansprüche, wobei das Metalloxid OM1 mit einem zweiten Metalloxid OM2 der Formel M_{c}Oₖ kombiniert wird, wobei:
M für ein Übergangsmetall steht;
c eine ganze Zahl im Bereich von 1 bis 3 ist; und
k eine Dezimalzahl im Bereich von 0,1 bis 4 ist,
und OM2 vorzugsweise die Formel CuO aufweist.

6. Verwendung nach Anspruch 5, wobei das Molverhältnis der Menge von Metalloxid OM1 zu der Menge von Metalloxid OM2 größer oder gleich 1, vorzugsweise größer oder gleich 2, weiter bevorzugt größer oder gleich 3, noch weiter bevorzugt größer oder gleich 4 und noch besser größer als 4 ist.

7. Verwendung nach einem der vorhergehenden Ansprüche, wobei die festen Teilchen, die das mindestens eine Metalloxid OM1 umfassen, durch Flammenspraypyrolyse erhalten werden oder erhalten werden können.

8. Verwendung nach einem der vorhergehenden Ansprüche zur Verhinderung, Verringerung und/oder Beseitigung der Ablagerungen von Proteinen und Fettsubstanzen, die vom Körper abgesondert werden, insbesondere Hauttalgablagerungen.

9. Verfahren zur kosmetischen Behandlung von Keratinmaterialien oder der Oberfläche eines Gegenstands in Kontakt mit den Keratinmaterialien, bei dem man:
(1) auf die Materialien bzw. die Oberfläche eine kosmetische Zusammensetzung aufbringt, die feste Teilchen, die mindestens ein Metalloxid OM1 der Formel AₐB_{b}Oₙ₋ₓ umfassen, gemäß einem der Ansprüche 1 bis 4 und 7 umfasst; dann
(2) die Materialien bzw. die Oberfläche natürlichem oder künstlichem Licht aussetzt.

10. Verfahren nach Anspruch 9, **dadurch gekennzeichnet, dass** die in Schritt (1) verwendete kosmetische Zusammensetzung ferner mindestens ein Metalloxid OM2 der Formel M_{c}Oₖ gemäß einem der Ansprüche 5 und 6 umfasst, das in den Teilchen, die das mindestens eine Metalloxid OM1 umfassen, oder in anderen festen Teilchen vorliegt.

11. Verfahren nach Anspruch 9 oder 10, **dadurch gekennzeichnet, dass** der Gehalt an Metalloxid OM1 in der kosmetischen Zusammensetzung im Bereich von 0,4 bis 40 Gew.-%, vorzugsweise von 0,5 bis 20 Gew.-%, noch besser von 1 bis 10 Gew.-% und noch weiter bevorzugt von 1,5 bis 5 Gew.-%, bezogen auf das Gesamtgewicht der Zusammensetzung, liegt.

12. Verfahren nach einem der Ansprüche 9 bis 11, **dadurch gekennzeichnet, dass** der Gehalt an Metalloxid OM2 im Bereich von 0,1 bis 10 Gew.-%, vorzugsweise von 0,15 bis 5 Gew.-% und noch besser von 0,25 bis 1,5 Gew.-%, bezogen auf das Gesamtgewicht der Zusammensetzung, liegt.

13. Verfahren zur kosmetischen Behandlung von Keratinmaterialien, bei dem man:
(1) die Materialien mit einem Artikel, der feste Teilchen, die mindestens ein Metalloxid OM1 der Formel AₐB_{b}Oₙ₋ₓ umfassen, gemäß einem der Ansprüche 1 bis 4 und 7 umfasst, in Kontakt bringt; dann
(2) den Artikel natürlichem oder künstlichem Licht aussetzt.

14. Verfahren nach Anspruch 13, **dadurch gekennzeichnet, dass** der in Schritt (1) verwendete Artikel ferner mindestens ein Metalloxid OM2 der Formel M_{c}Oₖ gemäß einem der Ansprüche 5 und 6 umfasst, das in den Teilchen, die mindestens ein Metalloxid OM1 umfassen, oder in anderen festen Teilchen vorliegt.

15. Verfahren nach einem der Ansprüche 9 bis 14, **dadurch gekennzeichnet, dass** Schritt (2) durch Lichtbestrahlung unter Verwendung einer Lichtquelle, die eine oder mehrere elektromagnetische Wellen mit einer Wellenlänge zwischen 200 nm im Ultraviolettbereich und 3000 nm im Infrarotbereich emittiert, und vorzugsweise unter Verwendung einer LED-Lampe, die Strahlung mit einer Wellenlänge im sichtbaren Bereich emittiert, durchgeführt wird.

## Revendications

1. Utilisation, pour prévenir, réduire et/ou éliminer les dépôts de composés organiques à la surface des matières kératiniques, notamment humaines, ou à la surface d'objets en contact avec lesdites matières kératiniques, de particules solides comprenant au moins un oxyde métallique OM1 de formule AₐB_{b}Oₙ₋ₓ, dans laquelle :
A désigne un métal choisi parmi le bismuth, le calcium, le sodium, le lanthane, le baryum, le cuivre, l'étain, le magnésium, le zinc et l'argent;
B désigne un métal choisi parmi le tungstène, le vanadium, le gallium, le niobium et le strontium;
a est un nombre entier compris dans la gamme allant de 1 à 4 ;
b est un nombre entier compris dans la gamme allant de 1 à 10 ;
n est un nombre entier compris dans la gamme allant de 3 à 6 ; et
x est un nombre décimal inférieur à n, compris dans la gamme allant de 0,1 à 5.

2. Utilisation selon la revendication 1, dans laquelle A est choisi parmi le bismuth et le baryum, et de préférence A désigne le bismuth.

3. Utilisation selon l'une quelconque des revendications précédentes, dans laquelle B est choisi parmi le tungstène et le vanadium, et de préférence B désigne le tungstène.

4. Utilisation selon l'une quelconque des revendications précédentes, dans laquelle l'oxyde OM1 a pour formule Bi₂WO₆₋ₓ, avec x un nombre décimal compris dans la gamme allant de 0,2 à 1,5, de préférence de 0,3 à 1 ; et mieux encore x est supérieur ou égal à 0,5 et strictement inférieur à 1.

5. Utilisation selon l'une quelconque des revendications précédentes, dans laquelle l'oxyde métallique OM1 est combiné à un second oxyde métallique OM2 de formule M_{c}Oₖ, dans laquelle :
M désigne un métal de transition ;
c est un nombre entier compris dans la gamme allant de 1 à 3 ; et
k est un est un nombre décimal compris dans la gamme allant de 0,1 à 4,
et de préférence OM2 a pour formule CuO.

6. Utilisation selon la revendication 5, dans laquelle le rapport molaire de la quantité d'oxyde métallique OM1 sur la quantité d'oxyde métallique OM2 est supérieur ou égal à 1, de préférence supérieur ou égal à 2, plus préférentiellement supérieur ou égal à 3, encore plus préférentiellement supérieur ou égal à 4, et mieux encore supérieur à 4.

7. Utilisation selon l'une quelconque des revendications précédentes, dans laquelle les particules solides comprenant ledit au moins un oxyde métallique OM1 sont obtenues, ou susceptibles d'être obtenues, par pyrolyse par projection de flamme.

8. Utilisation selon l'une quelconque des revendications précédentes, pour prévenir, réduire et/ou éliminer les dépôts de protéines et de corps gras sécrétés par l'organisme, et en particulier les dépôts de sébum.

9. Procédé de traitement cosmétique des matières kératiniques ou de la surface d'un objet en contact avec les matières kératiniques, comprenant :
(1) l'application sur lesdites matières ou sur ladite surface d'une composition cosmétique comprenant des particules solides comprenant au moins un oxyde métallique OM1 de formule AₐB_{b}Oₙ₋ₓ telles que définies dans l'une des revendications 1 à 4 et 7; puis
(2) l'exposition desdites matières ou de ladite surface à la lumière naturelle ou artificielle.

10. Procédé selon la revendication 9, **caractérisé en ce que** la composition cosmétique employée à l'étape (1) comprend en outre au moins un oxyde métallique OM2 de formule M_{c}Oₖ tel que défini dans l'une des revendications 5 et 6, présent dans lesdites particules comprenant au moins un oxyde métallique OM1 ou dans des particules solides distinctes.

11. Procédé selon l'une quelconque des revendications 9 et 10, **caractérisé en ce que** la teneur de l'oxyde métallique OM1 dans la composition cosmétique va de 0,4 à 40% en poids, de préférence de 0,5 à 20% en poids, mieux encore de 1 à 10% en poids, et encore plus préférentiellement de 1,5 à 5% en poids, par rapport au poids total de la composition.

12. Procédé selon l'une quelconque des revendications 9 à 11, **caractérisé en ce que** la teneur de l'oxyde métallique OM2 va de 0,1 à 10% en poids, de préférence de 0,15 à 5% en poids, et mieux encore de 0,25 à 1,5% en poids, par rapport au poids total de la composition.

13. Procédé de traitement cosmétique des matières kératiniques, comprenant :
(1) la mise en contact desdites matières avec un article comprenant des particules solides comprenant au moins un oxyde métallique OM1 de formule AₐB_{b}Oₙ₋ₓ telles que définies dans l'une des revendications 1 à 4 et 7; puis
(2) l'exposition dudit article à la lumière naturelle ou artificielle.

14. Procédé selon la revendication 13, **caractérisé en ce que** l'article employé à l'étape (1) comprend en outre au moins un oxyde métallique OM2 de formule M_{c}Oₖ tel que défini dans l'une des revendications 5 et 6, présent dans lesdites particules comprenant au moins un oxyde métallique OM1 ou dans des particules solides distinctes.

15. Procédé selon l'une quelconque des revendications 9 à 14, **caractérisé en ce que** l'étape (2) est mise en oeuvre par photoirradiation, au moyen d'une source de lumière émettant une ou plusieurs onde(s) électromagnétique(s) de longueur d'onde comprise entre 200 nm dans le domaine de l'ultra-violet et 3000 nm dans le domaine de l'infrarouge, de préférence au moyen d'une lampe de type LED émettant un rayonnement de longueur d'onde dans le domaine visible.
